(19) Europäisches Patentamt
European Patent Office
Office européen des brevets

(11) Publication number: 0 310 409 A2

# (12) EUROPEAN PATENT APPLICATION

(21) Application number: 88309085.4

(22) Date of filing: 30.09.88

(51) Int. Cl.[4]: C 07 C 177/00
// A61K31/557

(30) Priority: 02.10.87 US 104713 25.01.88 US 147839

(43) Date of publication of application:
05.04.89 Bulletin 89/14

(84) Designated Contracting States:
AT BE CH DE ES FR GB GR IT LI LU NL SE

(71) Applicant: Kawate, Tsuneyoshi
502 King of Prussia Road
Radnor Pennsylvania 19087 (US)

Ohnishi, Tsuyoshi
502 King of Prussia Road
Radnor Pennsylvania 19087 (US)

(72) Inventor: Ohnishi, Tsuyoshi
502 King of Prussia Road
Radnor Pennsylvania 19087 (US)

(74) Representative: Shaw, Laurence
George House George Road
Edgbaston Birmingham B15 1PG (GB)

## (54) Prostaglandin derivatives.

(57) Hydrophobic esters of prostaglandin, eg. the acetoxymethyl or acetoxyethyl ester of prostaglandin $A_1$ or prostaglandin $E_1$ are useful in the prevention or treatment of disorders caused by an increase in intracellular calcium concentration. Methods of preparing the esters and pharmaceutical compositions containing them are disclosed.

EP 0 310 409 A2

**Description**

## PROSTAGLANDIN DERIVATIVES

The invention relates to hydrophobic derivatives of prostaglandins, more particularly hydrophobic esters.

It is well documented that ischemic, physical, chemical and biological injuries cause an increase of intracellular calcium concentration in the injured cells, which in turn activates endogenous phospholipases and proteases. These enzymes not only attack cell membranes causing injury, but also initiate reactions whose metabolites could cause thrombosis, inflammation, allergy, ulcer and immune deficiency.

It is one object of the invention to provide derivatives of prostaglandin useful in the treatment or prevention of such conditions.

The invention is based on the realisation that certain derivatives of prostaglandin can protect cells by (a) chelating intracellular calcium ions, (b) inhibiting phospholipase and protease activities and, (c) inhibiting various endogenous enzymes which are involved in the cascade of reactions. The compounds were also found to protect organs of chronic alcoholic subjects.

Accordingly in one aspect, the invention provides a hydrophobic ester of a prostaglandin. Preferably the ester is an acetoxyalkyl ester, in which the alkyl group has a low number of carbon atoms; specifically preferred are the acetoxymethyl and acetoxyethyl esters. The prostaglandin is preferably prostaglandin $A_1$ or $E_1$.

In another aspect, the invention provides a method of making a hydrophobic ester as defined, the method comprising subjecting a prostaglandin to esterification, preferably by reaction with a halide, eg. bromide, of the desired ester.

In yet another aspect, the invention provides a pharmaceutical composition comprising a therapeutically effective amount of a hydrophobic ester as defined, and a pharmaceutical carrier or diluent.

The carrier or diluent may be liquid or solid and of known type.

The ester derivatives can be administered in a variety of ways, including orally, subcutaneously, intramuscularly, intravenously or topically. Preferred rates of administration (in mg/kg body weight/day) are about 10 for oral administration; about 6 for subcutaneous administration and about 2 for intravenous administration. The esters enter cells through the membranes because they have high affinity to lipids. They are decomposed inside the cells by cellular enzymes to become acid form molecules. Since the acid form molecules are less lipid soluble, they tend to stay inside the cells.

Compared to acid form compounds, the esters were found to have the following properties.

a) high solubility through cell membranes and easily transported into cells;

b) they are decomposed to acid form molecules by endogenous esterase enzymes;

c) the released acid form molecules are less soluble in a lipid phase so that they will stay inside the cell;

d) since the acid form molecules keep accumulating within the cells, their intracellular concentration will become much higher than the extracellular concentration of ester. This makes the administration of esters much more efficacious than that of acid form molecules;

e) the acid form molecules can chelate calcium, which protects injured cells from calcium toxicity;

f) the acid form molecules inhibit the endogenous phospholipase $A_2$ activity, thereby inhibiting the formation of arachidonic acid which is known to initiate reactions producing prostaglandins, prostacyclines, leukotrienes and lypoxins, which are known to have high biological activities to cause pathologic conditions such as thrombosis, inflammation, allergy, ulcer and immune deficiency; and

g) the acid form molecules can directly inhibit various endogenous enzymes involved in these reactions, thus protecting the cells from injury and degradation.

The alkyl group of the acetoxyalkyl ester preferably has from 1 to 6 carbon atoms in the alkyl group. As indicated, preferred are the acetoxymethyl and acetoxyethyl. The protaglandin may be $A_1$ or $E_1$, but it can also be $B_1$ or dehydro (keto) prostaglandin $A_1$, $B_1$ or $E_1$, the acetoxymethyl bromide, acetoxyethyl bromide or ethyl bromo acetate.

In order that the invention may be well understood, it will now be described by way of illustration only with reference to the following examples and the accompanying drawings, which relate to information in the examples.

EXAMPLE I

Preparation

0.1 g of prostaglandin $A_1$ or prostaglandin $E_1$ was dissolved in 10ml ethanol and added to 1.2g crushed solid KOH in a 50ml flask. The mixture was rotated on a reflux apparatus at 70° C for three hours. Ten ml of water was added to make the final base concentration 1 N KOH in 50% alcohol and the mixture was refluxed in an oil bath at 74° C for an additional six hours. The reaction mixture was cooled to room temperature, 11ml isobutanol was added and the pH adjusted to 3 with about 7.5ml 2.3 N $HClO_4$. The precipitate formed was allowed to settle, the supernatant decanted and the precipitate washed with $H_2O$ and a small amount of alcohol until white. The precipitate was discarded and the coloured supernatant and washing combined with an additional 20ml $H_2O$, 10ml of isobutanol and extracted at pH 3. The clear $H_2O$ layer was discarded. The red isobutanol layer was washed twice with 10ml of water and finally extracted with 25ml of 0.1 M freshly prepared $NaHCO_3$ (pH

8.5). 10ml of isobutanol was added to the separated $NaHCO_3$ solution which contains oligomers, and the pH adjusted to 3 with 2.7 ml of 1N HC1. The active components were than all extracted into the isobutanol layer which was washed with $H_2O$, dried and flash evaporated to yield a compound which is defined as MR-256. This compound is in an acid form. The molecular weight of this compound as determined by vapour pressure osmometry is about 2,200 suggested that the major component of this compound may be hexamers, although trimer, tetramer, pentamer and heptamer are also contained. The yield was 70-80%.

50mg of MR-256 was mixed with 0.5ml of ethyldiisopropylamine, 0.1 ml chloroform, and 2 ml of isobutanol. Then 0.2 ml of acetoxymethyl bromide or acetoxyethyl bromide was added and the mixture was stirred in a stoppered flask for 20h. The solution was evaporated in vacuo, the residue taken up in benzene, filtered to remove the undesired ethyldiisopropylammonium bromide, and re-evaporated. The residue was extracted with diethyle ether several times. The extracts were combined and evaporated in vacuo overnight. The yield of the ester form is 60-70%. This ester-form is denoted as MR-356 for identification purpose. MR-356 is not water-soluble, but soluble in ethanol.

Compounds MR-256 and MR-356 were developed on a silica gel thin layer chromatography plate using a mixture of benzene and methanol (ratio 2:1). MR-256 remained at the origin, whereas the Rf value for MR-356 was 0.69. This result confirms that the ester-form is much more hydrophobic than the parent acid compound.

A high dose of MR-256 or MR-356 (70mg/kg body weight) was injected (i.p.) into five mice each. No toxicity was observed; the animals behaved normally.

Example II

Protective effects in heart Ischemia

a) Protection of myocytes:

Myocytes (isolated cardiac cells) were prepared from the rat heart according to the method of Hohl C, Alatschuld R A and Brieley G P, (Arch Biochem Biophys 221:197-205, 1983). About 60 to 70% of myocytes were in rod-shape form (a rod-shape indicates intactness of isolated cardiac cells). When these myocytes were kept under nitrogen for 1 hour, about half of the rod-shaped cells were irreversibly damaged and turned into round-shaped cells. MR-356 was added to the suspension before the nitrogen exposure and it was observed that the formation of round-shaped cells was inhibited. At concentrations less than 1 uM, the drug protected myocytes from ischemic damage as indicated by the preservation of rod-shaped cells and as shown graphically in Figure 1.

(b) Protection of isolated perfused rat heart from ischemia

An isolated rat heart was mounted by tieing the aorta to a catheter in the Langendorff model shown in Figure 2. A modified Krebs-Henseleit solution (117 mM NaCl, 6mM KCl, 2.5 mM $CaCl_2$, 1.2 mM, $MgC1_2$, 2.4mM phosphate, 24mM $NaHC0_3$, 5mM glucose; bubbled with 95% $O_2$-5% $CO_2$ gas; temperature 370; pH 7.4) was circulated by a pump (flow rate: 15ml/min). The heart was stimulated 3 beats/seconds by electric pulses (10V; 10msec duration). This system supported the beating heart for more than several hours. The left ventricular output (P) was measured by inserting a catheter with a small latex balloon attached at the tip, and by attaching the catheter to a Statham pressure transducer P23Gb. After equilibration, the flow of Krebs-Henseleit solution was stopped for 15 minutes. If no drug was added, the heart could hardly beat after the flow was restarted (the cardiac output recovered only to 10-15% of the original level; see the graph of Figure 3A). However, if 3.8uM MR-356 was added before restarting the flow, the heart recovered more than 75% - see the graph of Figure 3B. Thus, the drug was able to protect the heart from the "reflow" damage. Protective effects of MR-356 and MR-256 are shown in the block diagram of Figure 4(A).

(c) Suppression of CPK level:

Another method of measuring protective effect of drugs is to assay the creatine phosphokinase activity (CPK activity) of the circulating solution. When the heart was damaged by ischemia, the CPK activity appeared into the circulating solution after the reflow (see no-treat or control result in Figure 4(B)). However, if drugs were added before the reflow, the level of CPK activity was much reduced as shown in the other block diagrams of Figure 4B.

EXAMPLE III

Effect of drugs on global liver ischemia

Rats weighing 150 to 175 grams were used. Under enflurane anesthesia, a microvascular clamp was placed to occlude the portal vein as well as the hepatic artery, which supplied blood to the left lateral and median lobes of the liver. The rats were subjected to ischemic conditions ranging from 1 to 3 hours, after which reperfusion of the liver is allowed by removing the clamp. Twenty four hours later, blood samples were drawn for the SGPT (serum glutamate-pyruvate transaminase) assay. As shown in the graph of Figure 5, after 2 or 3 hours ischemia the SGPT level steeply increased (to levels of 2,000 to 3,000 U/dl) from the control level which is about 100U/dl. Using 2 hours of ischemia, we tested the effects of the drugs as shown in the graphs of Figure 6. Both Mr-256 and Mr-356 suppressed the increase of SGPT levels by 60 -70%. The maximum effect was observed around 3 to 5 mg/kg body weight.

EXAMPLE IV

Protection of kidney ischemia

Experiments were performed on Sprague-Dawley male rats. Both kidneys were exposed through a mid-line incision and the left renal artery was occluded with a microvascular clip to induce a 70 minutes ischemia. The right kidney was then removed, and left renal blood flow was re-established; the incision was closed and the rat was allowed to recover. In the drug-treated groups, intravenous infusion of MR-356 (5mg/kg) was started 30 minutes prior to renal artery occlusion and continued throughout the ischemia time and 30 minutes after the ischemia. The protective effect of the drug on the acute renal failure is shown in the block diagram of Figure 7. By 70 minute global ischemia, only 17% of untreated rats survived for 4 days. However, if the rats were given the drug, 83% were able to survive for 4 days.

EXAMPLE V

Effects of drugs on brain ischemia

(a) Surgical procedures:

Adults male Sprague-Dawley rats weighing 250-300 g were used to prepare focal ischemia model developed by Chen et al. (Stroke 17:738-743, 1986). Anesthesia was induced and maintained with halothane (1,0-2.5%) via a closely fitted facial mask. Bilateral common carotid arteries (CCA) were exposed gently and the right CCA was ligated in two placed by 4-0 silk suture. To expose the right middle cerebral artery (MCA) the temporal muscles were cut and retracted, then, a small temporal craniotomy (2x2mm) was performed with a microdrill. Under a microscope, the dura mater was opened with a 26 gauge needle, and the MCA was ligated by 10-0 suture with a square knot. Immediately, the left CCA was occluded by a microaneurysmal clip. After the operation, the animal was returned to its cage. One hour later, the left CCA clip was released in awake condition. The sham-operated control was produced in a similar procedure, except for the ligations and clipping of vessels. The operation was usually finished within 30 minutes.

b) Water content and ionic analysis

After fixed time intervals (1, 3 and 7 days), the animal was sacrificed and the excised cerebral hemisphere was placed in a preweighed crucible and measure and wet weight (WW) with a chemical balance (Mettler A E 100). The sample was then dried in an oven at 105° C until it reached a constant weight, which is defined as a dry weight (DW). The water content (% wet weight) was calculated (WW - DW)/WW.

The water content of the right hemisphere (ischemia side) was increased, showing that the brain edema developed. If drugs (3 mg/kg) were administered i.v. thirty minutes prior to the ischemia insult, the increase of water content was much reduced as shown in the block diagram of Figure 8.

(c) Motor performance test:

The disturbance of motor functions was evaluated by the total score obtained from the inclined plate test, balance beam test and prehensile test. These motor performance tests were designed to quantify the motor weakness in the global ischemia rats. The original method was modified to suit to the rat focal ischemia model employed in this study, whose motor deficits may be less severe than that of global ischemia (4-vessel occlusion, 20 min). Two trials were given to the animal, and the better score was chosen as its data. To lessen the factor of fatigue, the trial was carried out at intervals of a few minutes. In the inclined plate test, a 60 x 30 cm board covered with a thin rubber pad was used as a plate and was fixed at a designated angle (55°). The animal was placed at the highest position of the plate and the time which it could stay on the plate was measured until 30 seconds. In the balance beam test, a wooden rod, 70 cm long and 3.2 cm diameter, was positioned horizontally 60 cm above a thick sponge pad and the animal was left at the centre of rod. The time that the rat spent on the rod was recorded until 30 second. In the prehensile test, a Nylon rope, 70 cm long and 4mm in diameter, was stretched horizontally 60 cm above a thick sponge pad and the rat's forepaws were placed on the rope and then released. The time that the rat spent hanging on the rope was measured until 30 seconds. The scoring methods in these three tests were identical and defined as follows; 0:0 second; 1;1-10 seconds; 2:11-20 seconds; 3:21-30 seconds; 4:above 30 seconds. Therefore, the total motor score ranged from 0 to 12 points.

Figure 9 shows in block diagram from the motor function as determined by three tests (complete paralysis is indicated by motor score 0, and normal condition as 12). By focal ischemia, 3 days later, the animal lost about 50% of motor ability as indicated by the motor score of 7 in non-treated animals. In 7 days, animals recovered to approximately the score of 8. With these drugs given before ischemia (3mg/kg), the motor score obtained 7 days after the operation was about 11 indicating that animals were almost completely recovered (they may still have slight motor deficits).

(d) Passive avoidance test:

The apparatus comprised an illuminated large compartment (40x40cm) and a dark small compartment (10x10cm) interconnected by an opening (6x6cm). Grids made of metal conductors were placed at the bottom of the small compartment. The rat was placed on a fixed location in the large compartment and was allowed to explore the apparatus for 180 seconds. The time spent in the small compartment was recorded with a stop watch. Then, when the rat was in the small compartment, the opening was closed with a transparent shutter and the rat received a weak electric shock for 30 seconds (to a degree that the rat felt a slight pain). By this method, the rat had an experience of fear (learning) and passive avoidance reaction was established. The retention test was undertaken in the same manner as the first exploration 24 hours after the initial test.

After receiving the electric shock, normal rats remembered the pain and never went back to the dark room again (retention time was 0). However, in ischemia rats, their memory was disturbed; they went back into the dark room. The results of Figure 10(A) shows that ischemic rats spent approximately 120 seconds in the dark room (as shown by shadowed column of retention time).

When the drugs were administered before the ischemia, animals maintained better memory as shown in the results of Figure 10(b). The retention time was significantly decreased (to 40 to 50 seconds).

EXAMPLE VI

Spinal Cord Injury

In spinal cord injury, the primary injury is accompanied by immediate loss of nerve action potential and rapid ion movement. This is followed by secondary events which results in cell death in a matter of hours.

a) Experimental procedure:

A method developed by Wrathall's group (Wrathall et al, Exper Neurology 88:108-122, 1985; Noble L J and Wrathall J R, Exper Neurology 95:530-533, 1987) was used. Animals were anesthetised with nembutal (50mg/kg). Laminectomy was performed on the T-11 segment of the spinal cord. The skin wound was temporarily closed and the animal recovered from the effect of laminectomy. Several hours later, the animal was anesthetised with 1.5% halothane (air as carrier). The T-11 segment was again exposed and an impounder (made of plastic with a tip diameter of 2.4mm) was placed on the exposed dura. Then, a 10 gram weight was dropped from the height of 5cm onto the impounder. The impounder remained on the surface of the spinal cord for a few seconds, after which it was removed. The skin wound was sutured and closed. The animal was returned to the cage after regaining consciousness. The bladders were compressed twice daily to induce urination.

b) Assessment of motor recovery:

After contusion, animals were observed for four weeks during which time the recovery was quantified by rating the hind-limb walking ability according to the method originally used by Tarlov (Tarlov, I M Arch Neurol psychiatry 71:588-597, 1954) and modified by Wrathall et al. (Exper. Neurology 88:108-127, 1985). This is basically a scoring system for hind limb mobility ranging from 0 to 5. Zero indicates complete paralysis while a score of 5 indicates full recovery. As shown in Figure 11, measurements were taken from a group of rats receiving no treatment (control) and a group receiving drug treatment after injury. The drug was administered i.p. three times; 0.5, 6 and 12 hours after injury (each time 5 mg/kg body weight). As shown in the graph of Figure 11, both MR-256 and 356 improved the recovery, but MR-356 was more effective.

EXAMPLE VII

Sickle cell anaemia

Irreversibly sickled cells (ISC, which are irreversibly denatured sickle cells) have been produced by exposing sickle red blood cells to a repeated sickling-unsickling cycling in vitro and this can be used to determine the efficacy of drugs in protecting cell membranes (Ohnishi, Br J Haematol 55:665-671, 1983; Ohnishi et al Biochim Biophys Acta 886:119-129, 1986). When sickle cells were exposed to a sickling-unsickling cycling for 3 hours, about half of the cells were denatured as revealed by the formation of heavy, dehydrated cells in density gradient centrifugation. These heavy cells were collected, the amount measured, and the percentage of production calculated (Table 1). As shown in the Table below, when 13 uM of MR-356 was added, the formation of irreversibly denatured cells was remarkably inhibited. MR-256 was less effective; even with 40uM, formation of irreversibly denatured cells was not much inhibited (Table 2).

Example VIII

Malaria

Two different kinds of plasmodium were tested: Plasmodium Chabaudi and Plasmodium Vinckei. In the P Chabaudi experiment, the increase in parasitemia was measured after each mouse was infected by $10^6$ parasite-infected red blood cells. After 8 days, the parasitemia in the non-treated group was almost 30%. As shown in the graph of Figure 12, the effect of MR-256 was not significant, but the increase of parasitemia was almost completely inhibited by MR-356 (20 mg/kg per day). In case the of P Vinckei, the survival rate was also measured together with parasitemia. Since these parasites are so strong, they kill all mice in the end. As shown in the graph of Figure 13, infection by $10^6$ parasite-infected red blood cells killed mice in the control group in 6 1/2 days. However, with MR-356 (2 mg/kg per day), the survival time was prolonged, and 20% of animals survived. The rate of increase of parasitemia was also inhibited by MR-356 as shown in the figure. In P Vinckei experiments, MR-256 did not show any beneficial effect.

Table 1

Protective effect of MR-356 against the formation of irreversibly sickled cells caused by sickling-unsickling cycling (numbers indicate the percentages)

| | Drug during cycling | | | No cycling |
|---|---|---|---|---|
| | 0 | 13 | 40uM | 0 |
| Reversibly sickling cells | 52.3 | 93.1 | 100 | 100 |
| Irreversibly sickled cells | 47.7 | 6.9 | 0 | 0 |

Table 2

protective effect of MR-256 against the formation of irreversibly sickled cells caused by sickling-unsickling cycling (numbers indicate the percentages)

| | Drug during cycling | | | No cycling |
|---|---|---|---|---|
| | 0 | 13 | 40uM | 0 uM |
| Reversibly sickling cells | 55.7 | 64.6 | 74.8 | 100 |
| Irreversibly sickled cells | 44.3 | 35.4 | 25.2 | 0 |

**Claims**

1. A hydrophobic ester of a prostaglandin.
2. An acetoxyalkyl ester of aprostaglandin.
3. The acetoxymethyl or acetoxyethyl ester of a prostaglandin.
4. An ester according to any preceding Claim characterised in that the prostaglandin is prostaglandin $A_1$ or $E_1$.
5. A method of preparing a hydrophobic ester of a prostaglandin characterised by subjecting a prostaglandin to esterification to form a hydrophobic ester.
6. A method according to Claim 5 characterised by reacting the prostaglandin to reaction with a halide of the desired ester.
7. A method according to Claim 6 characterised in that the ester is an acetoxyalkyl ester and the halide is the bromide.
8. A method according to Claim 7 characterised in that the ester is the acetoxymethyl or acetoxyethyl ester.
9. A method to any of Claims 5 to 8 characterised in that the prostaglandin is prostaglandin $A_1$ or $E_1$.
10. A pharmaceutical composition characterised in that the composition comprises a pharmaceutical carrier or dilutent and an effective amount of one or more hydrophobic esters of a prostaglandin.
11. A composition according to Claim 10 characterised in that it comprises a dosage unit form for administration orally at the rate of 10mg/kg body weight/day or a dosage unit from for administration intravenously or intramusculary at the rate of 6mg/kg body weight/day.
14. A composition according to Claim 11 characterised in that it comprises a dosage unit from for administration intravenously at the rate of 2mg/kg body weight/day.

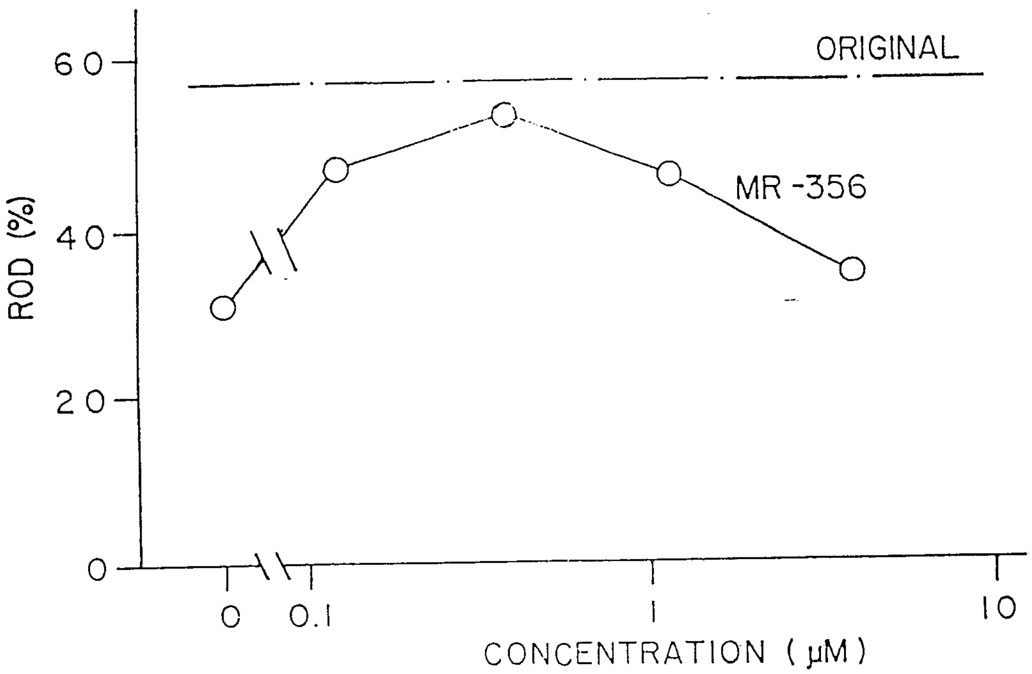


Fig. 1

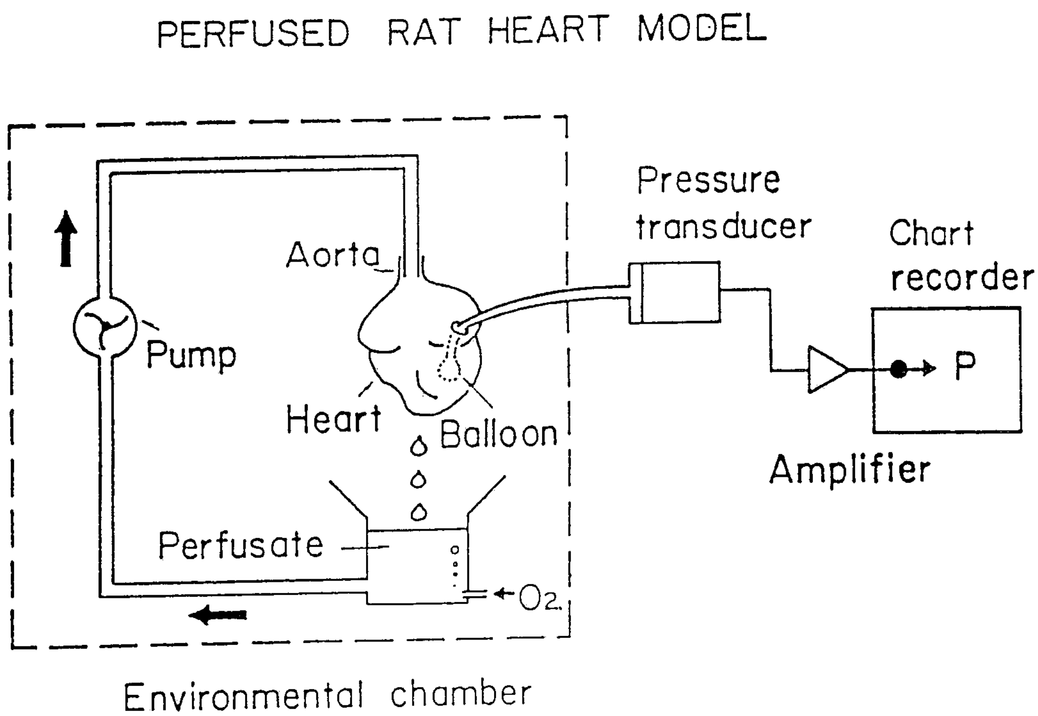
PERFUSED RAT HEART MODEL
Pressure transducer
Chart recorder
Aorta
Pump
Heart
Balloon
P
Amplifier
Perfusate
O2
Environmental chamber

Fig. 2.

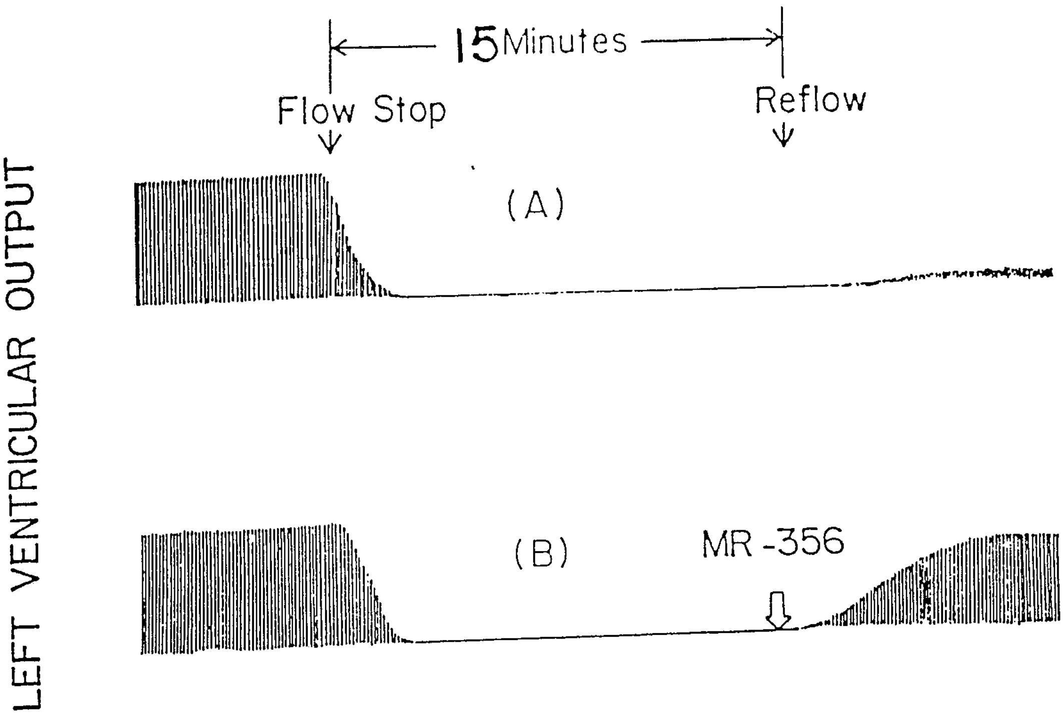
15 Minutes
Flow Stop
Reflow
(A)
LEFT VENTRICULAR OUTPUT
(B)
MR-356

Fig. 3

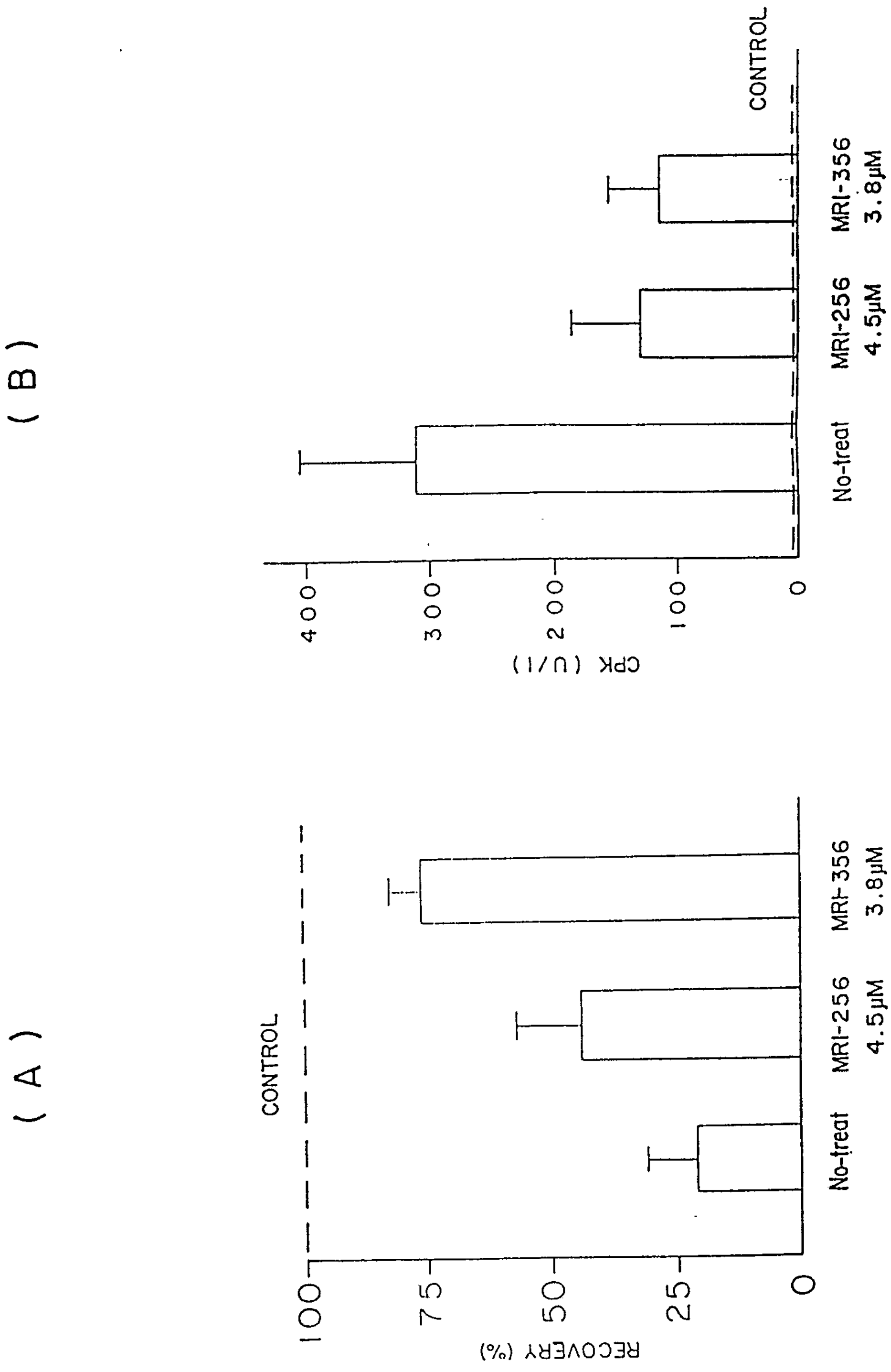

( A )
RECOVERY (%)
100
75
50
25
0
CONTROL
No-treat
MRI-256
4.5μM
MRI-356
3.8μM
( B )
CPK (U/l)
400
300
200
100
0
CONTROL
No-treat
MRI-256
4.5μM
MRI-356
3.8μM


Fig. 4

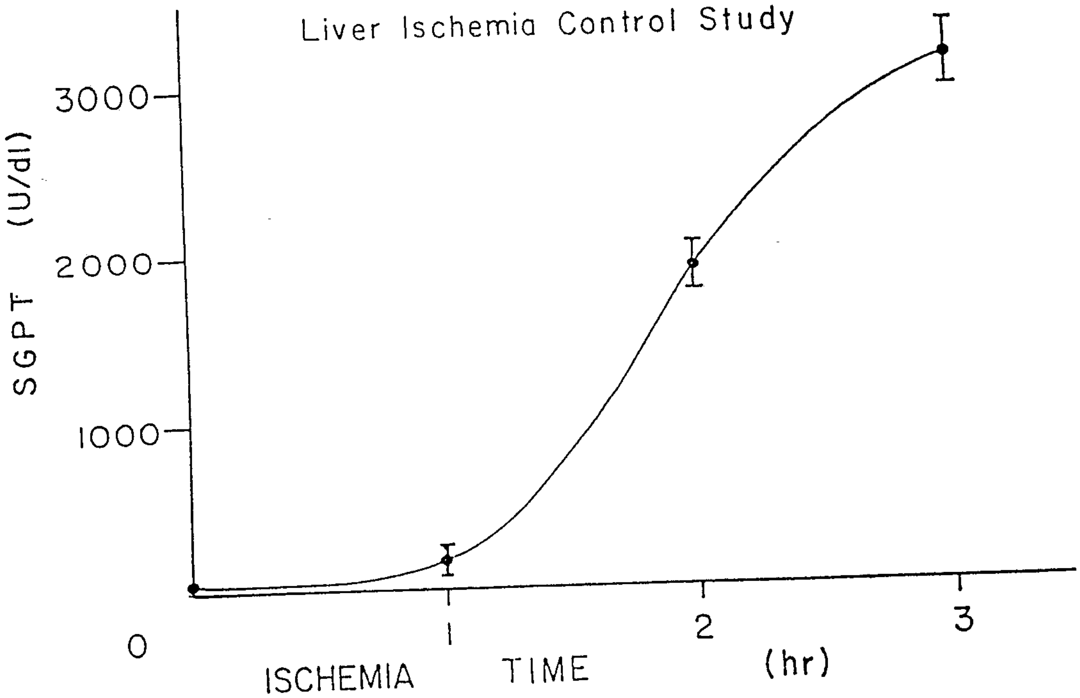
Liver Ischemia Control Study
3000
2000
1000
SGPT (U/dl)
0
1
2
3
ISCHEMIA TIME (hr)

Fig. 5

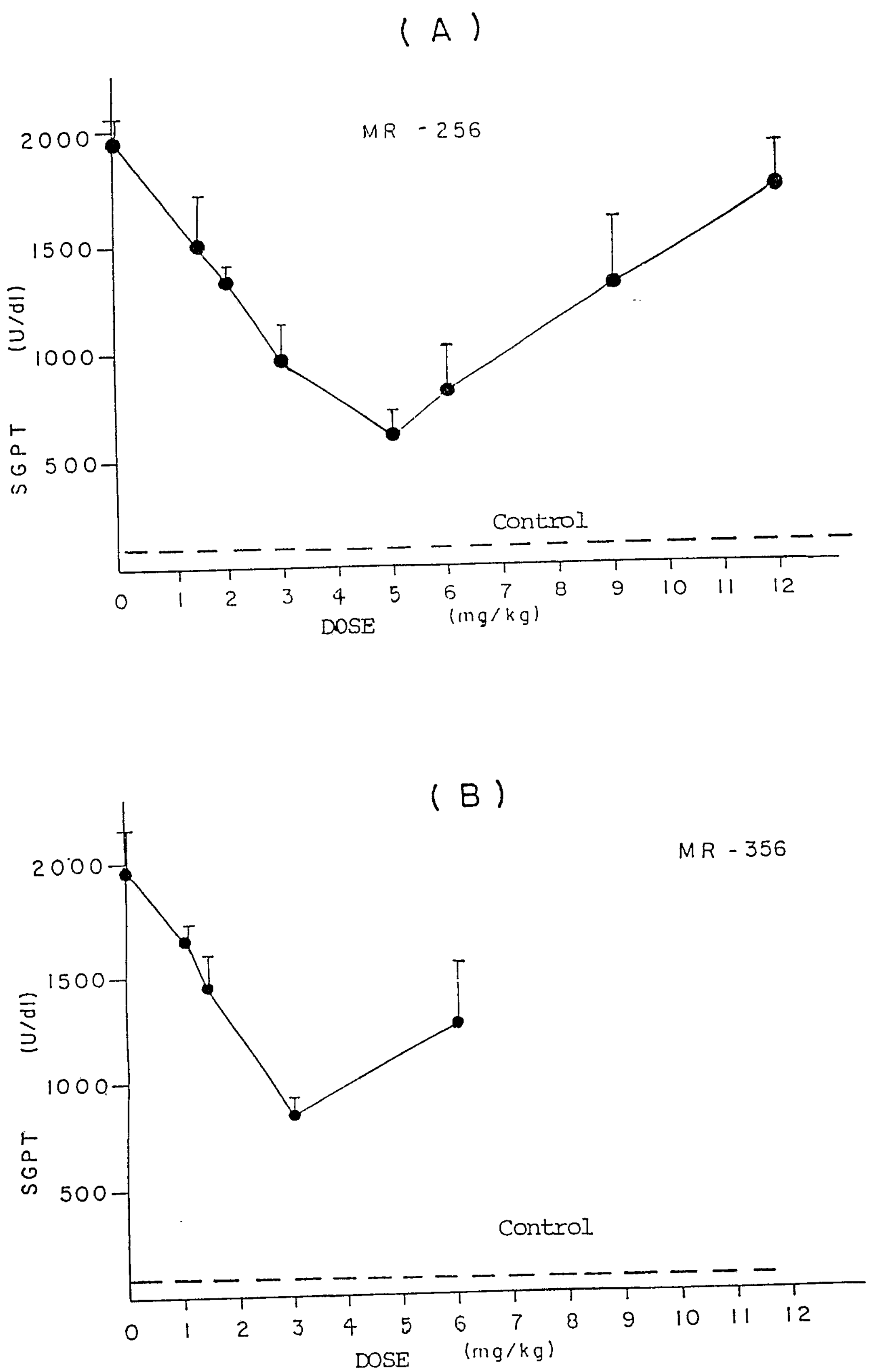
( A )
MR -256
2000
1500
1000
500
SGPT (U/dl)
Control
0
1
2
3
4
5
6
7
8
9
10
11
12
DOSE (mg/kg)
( B )
MR -356
2000
1500
1000
500
SGPT (U/dl)
Control
0
1
2
3
4
5
6
7
8
9
10
11
12
DOSE (mg/kg)

Fig. 6

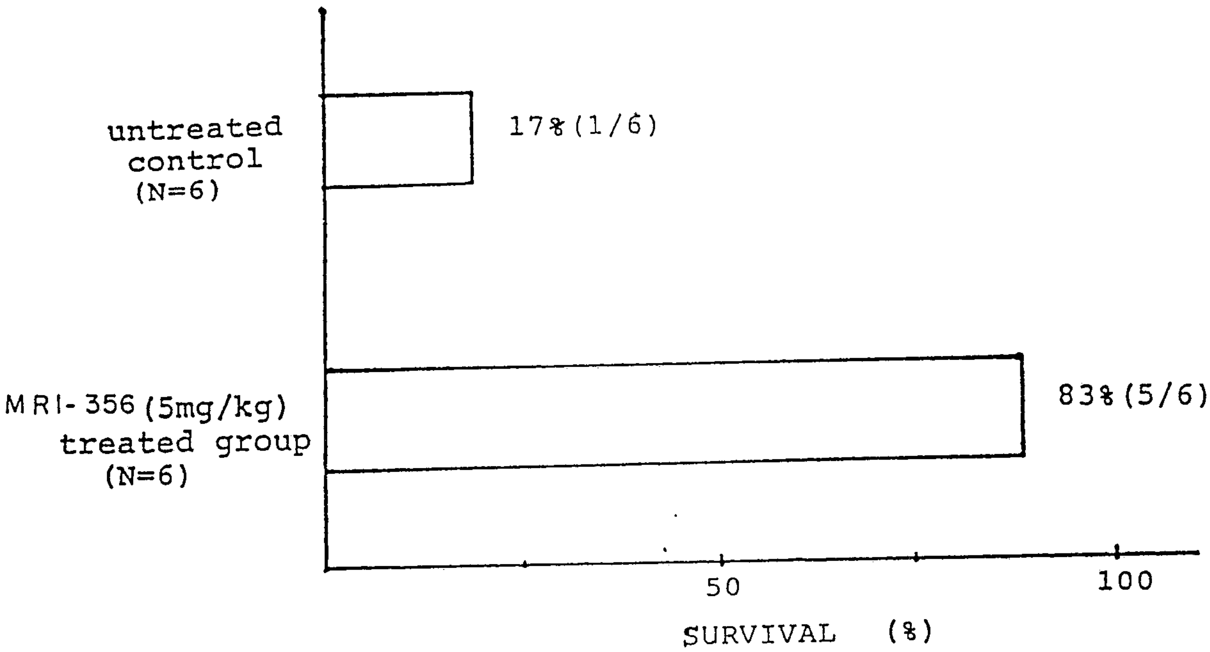
untreated
control
(N=6)
17% (1/6)
MRI-356 (5mg/kg)
treated group
(N=6)
83% (5/6)
50
100
SURVIVAL (%)

Fig. 7

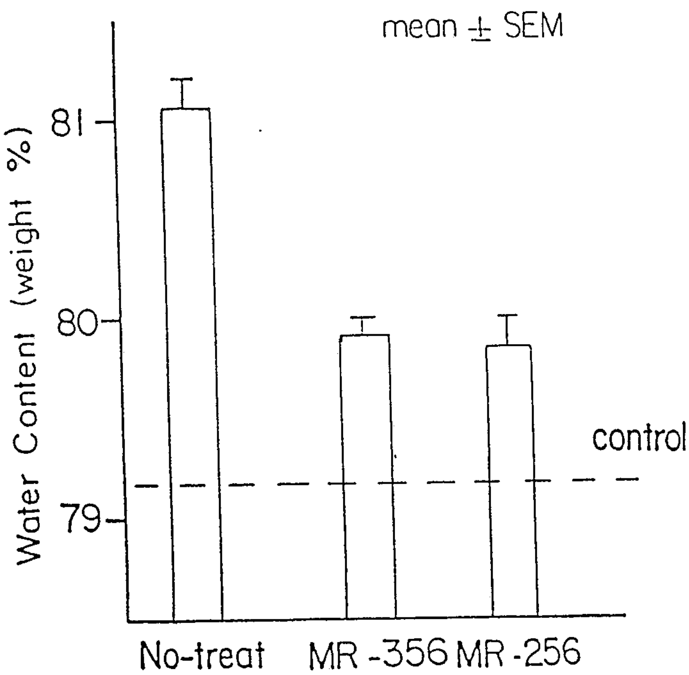
mean ± SEM
Water Content (weight %)
81
80
79
control
No-treat
MR -356
MR -256

Fig. 8

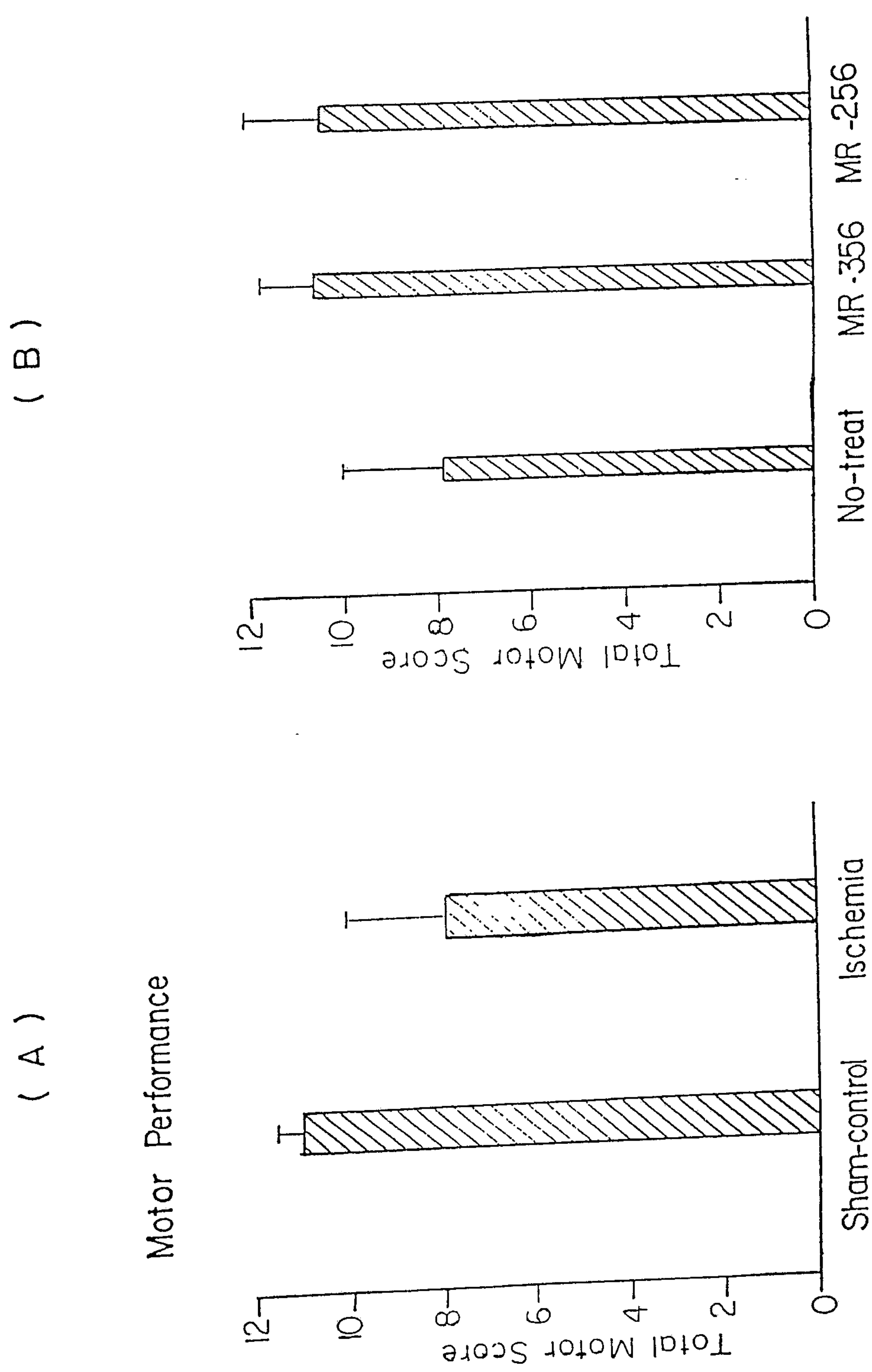
( A )
Motor Performance
Total Motor Score
0
2
4
6
8
10
12
Sham-control
Ischemia
( B )
Total Motor Score
0
2
4
6
8
10
12
No-treat
MR -356
MR -256

Fig. 9

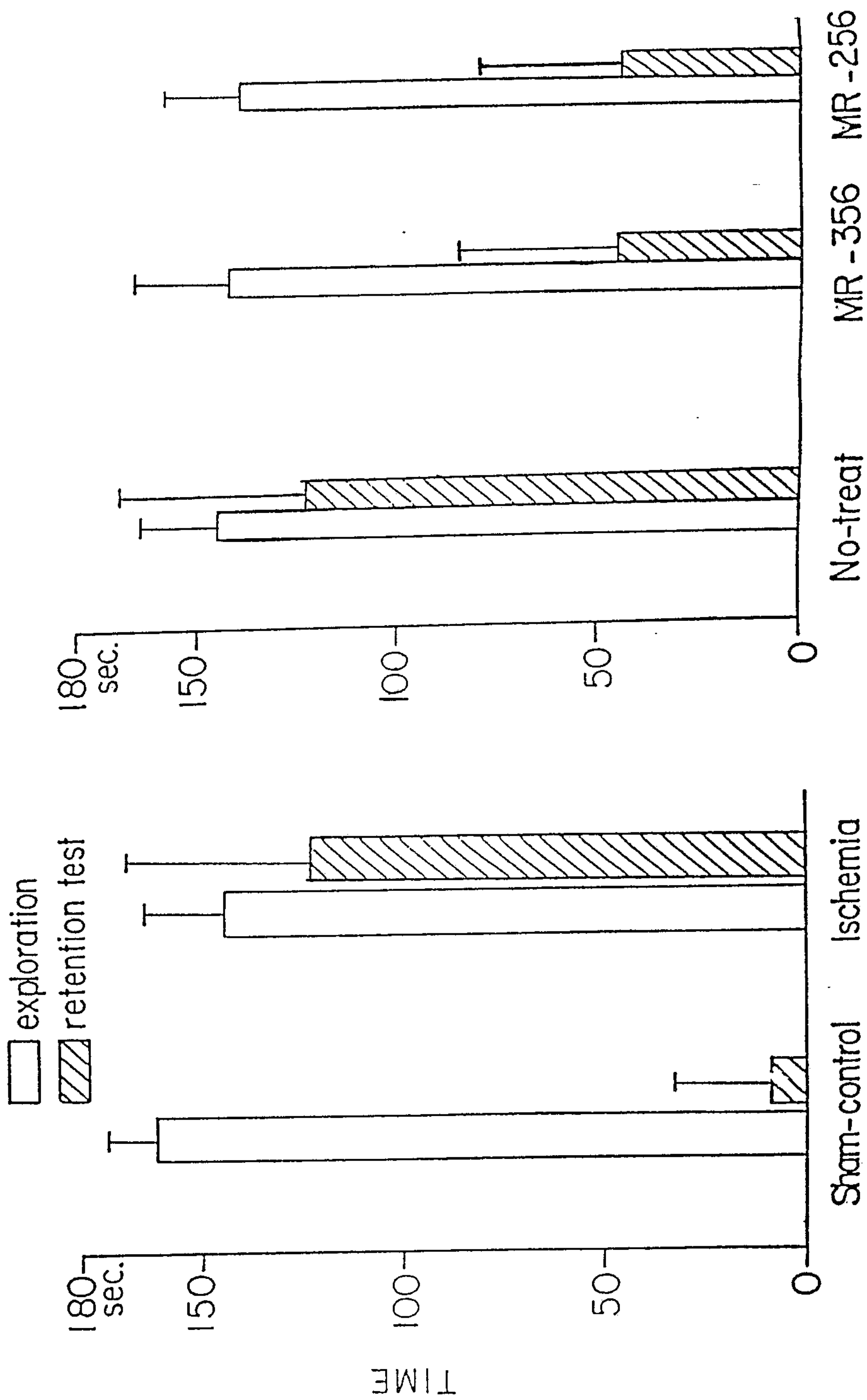
exploration
retention test
TIME
180 sec.
150
100
50
0
Sham-control
Ischemia
No-treat
MR -356
MR -256

Fig. 10 (A)

(B)

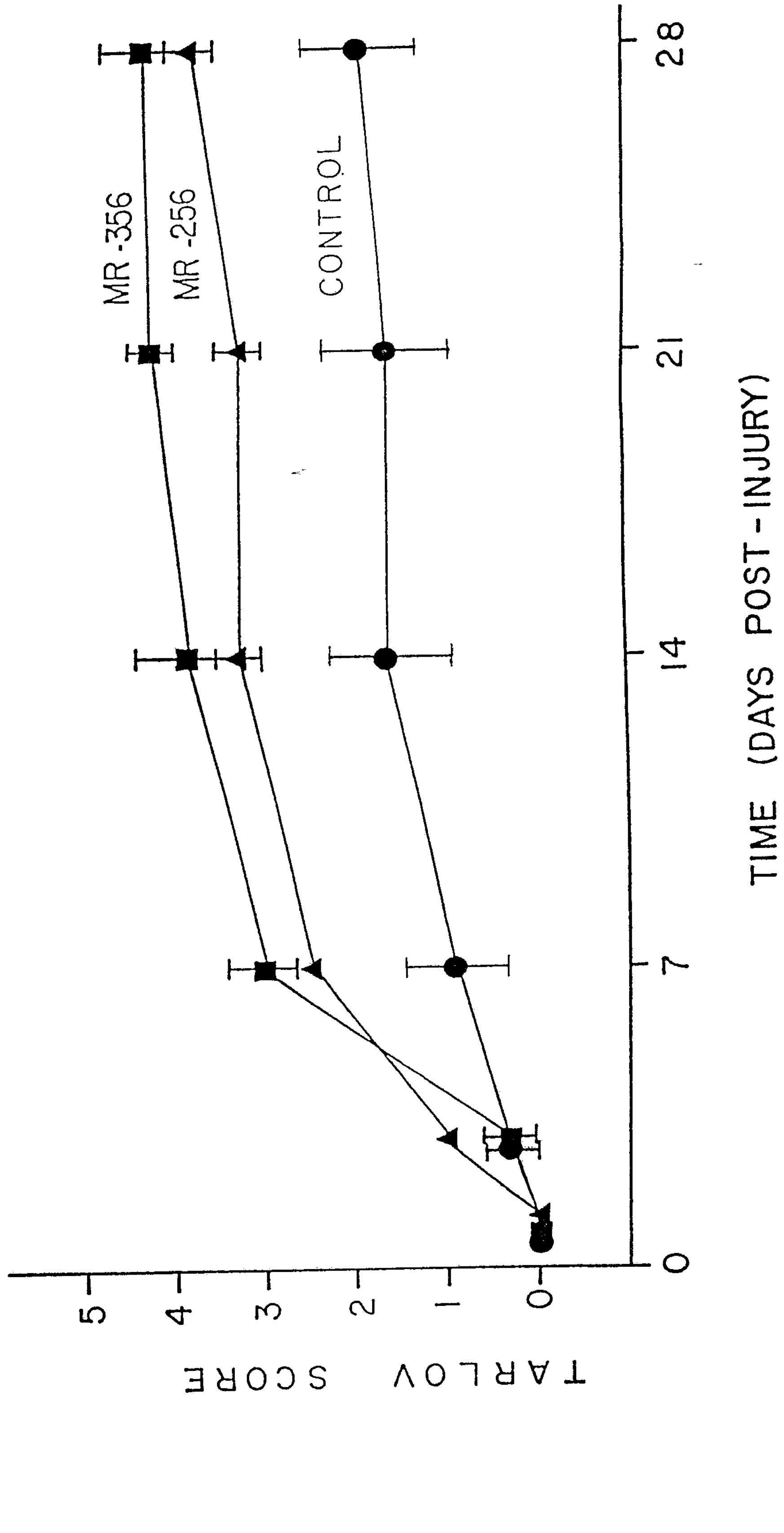
TARLOV SCORE
0
1
2
3
4
5
MR-356
MR-256
CONTROL
0
7
14
21
28
TIME (DAYS POST-INJURY)

Fig. 11

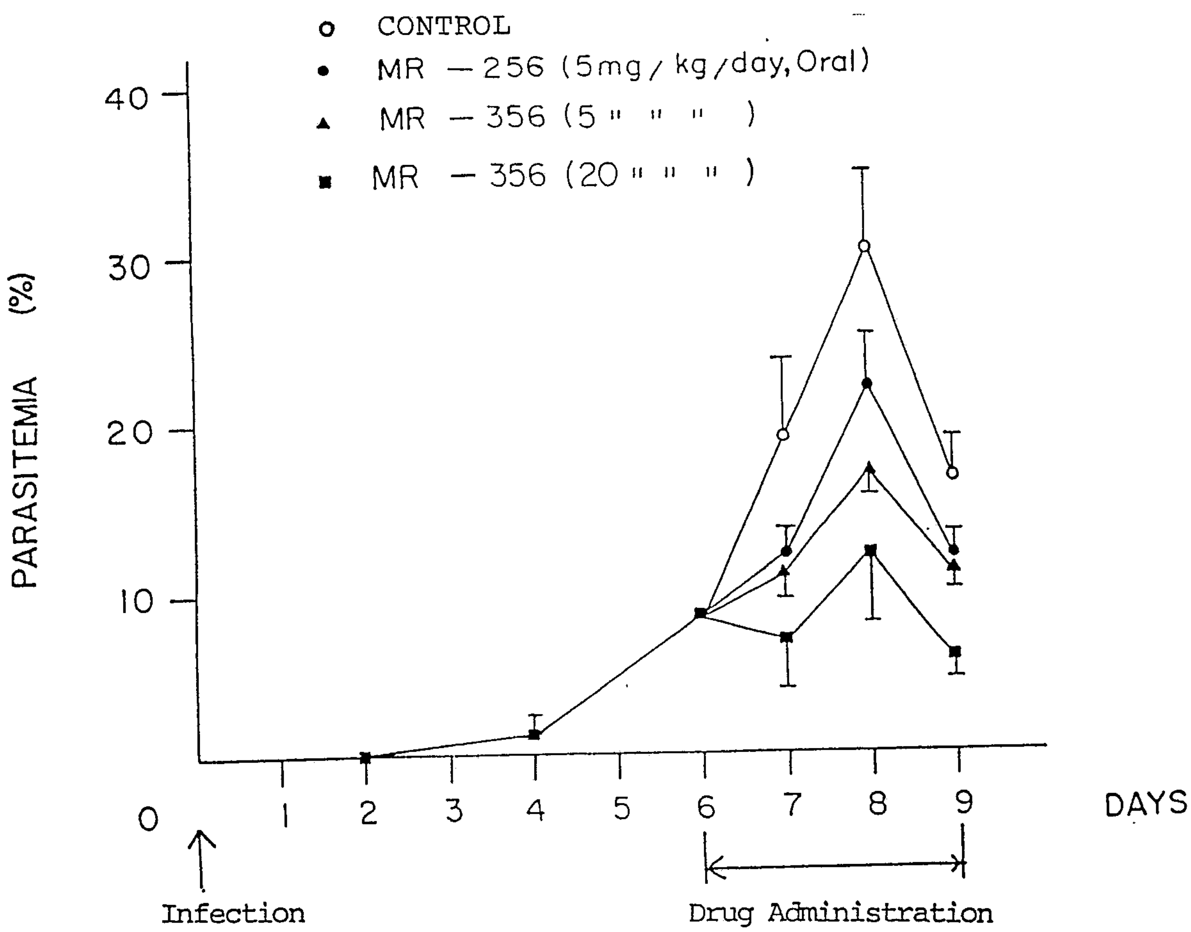

CONTROL
MR — 256 (5mg / kg / day, Oral)
MR — 356 (5 " " " )
MR — 356 (20 " " " )
40
30
20
10
PARASITEMIA (%)
0
1
2
3
4
5
6
7
8
9
DAYS
Infection
Drug Administration


Fig. 12.

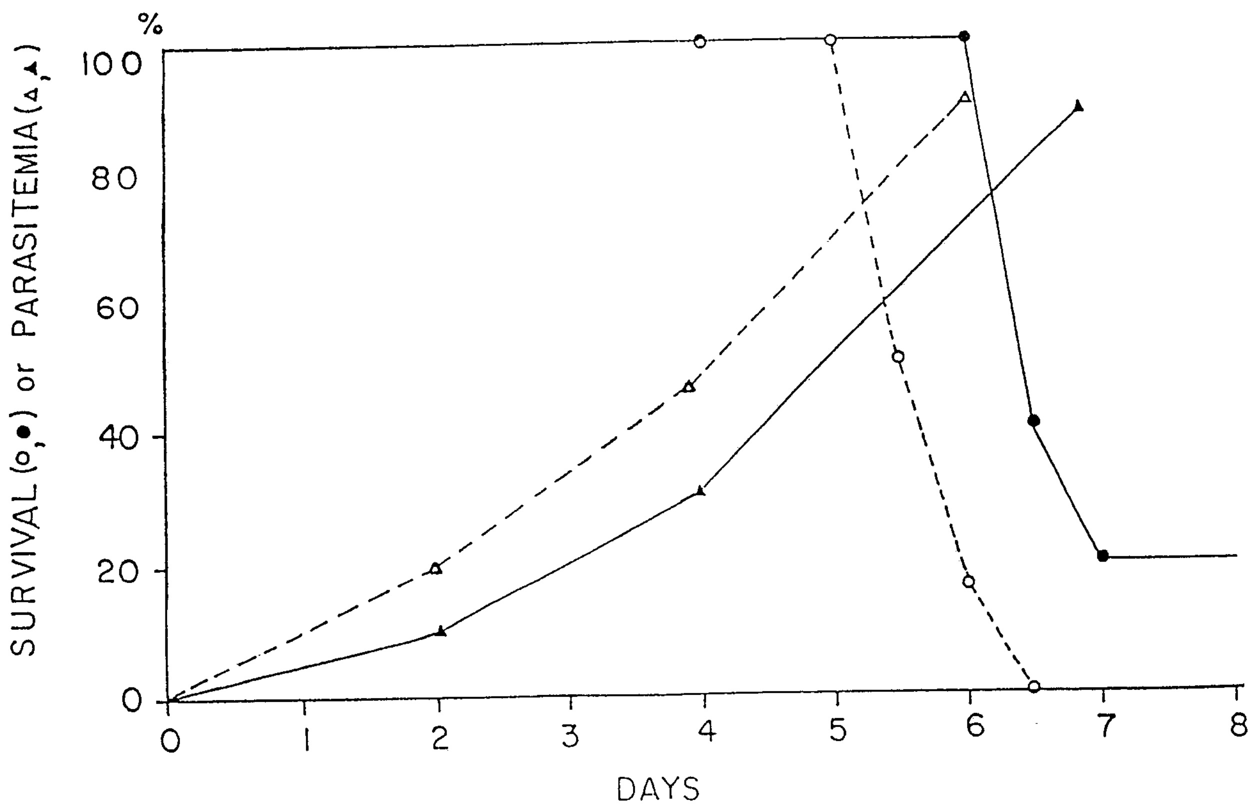


Fig. 13.